# EUROPEAN PATENT APPLICATION

(11) **EP 1 468 662 A1**
(43) Date of publication of application: **20.10.2004**
(21) Application number: 03076140.7
(22) Date of filing: 17.04.2003
(51) Int. Cl.: A61K 6/00

(54) **Polymerisable composition, method for polymerising and filling dental cavities**

(71) Applicant: Universiteit van Amsterdam, 1012 WX Amsterdam (NL)
(72) Inventor: The designation of the inventor has not yet been filed
(74) Representative: Prins, Adrianus Willem, Mr. Ir.

(57) **Abstract**

The present invention relates to a polymerisable composition, to a method for polymerizing said polymerisable composition and to a method for filling a dental cavity. Particularly the present invention makes it possible to reshape a filling material when said material is already partially cured.

## Description

The invention relates to a polymerisable composition and especially to such a composition to be used for medical applications, such as a resin based restorative material in dentistry. Additionally, the invention relates to a method for polymerising such a polymerisable composition. The invention also relates to a method for filling a dental cavity.

Polymerisable compositions to be used in medical applications and specifically in the field of dentistry are known *per se.* Usually, such a composition comprises an initiator which is capable of initiating polymerisation of the composition upon intake of a form of energy. This energy may for instance comprise heat, ultrasound or microwave radiation. Often, an initiator needs to receive a minimum amount of energy, generally referred to as the activation energy, before initiating the polymerisation reaction.

The polymerisation kinetics and the molecular structures of the polymers formed during polymerisation generally both depend at least partly on the initiation of the polymerisation. Within this context, the term "kinetics" is used for the relationship between reaction time and progress of the polymerisation reaction. Initiation itself, depends amongst others on the amount of energy applied to the composition. Once an initiator has received an amount of energy that is at least equal to the activation energy, that initiator will be activated.

Initiator molecules are usually divided into two groups namely free radical initiators and ionic initiators. Activation of an initiator molecule implies an initiating reaction and involves the breakdown of an initiator molecule in either radicals or ions, depending on the group the initiator molecule belongs to. The polymerisation reaction is triggered by the presence of the radicals and/or the presence of ions. The extend of polymerisation is, amongst other factors, dependent on the number of free radicals and/or the number of ions produced and is therefore dependent on the amount of initiators present in the composition. Often however, the polymerisable composition comprises enough initiators to ensure that once the initiator molecules are activated, polymerisation occurs to a predetermined extend of polymerisation which forms the final result of the polymerisation. As stated before, the polymerisable composition may be applied in a resin based restorative material as used in dentistry. Prior to the polymerisation this material is paste-like to allow for re-shaping or modelling the material in a required shape.

Polymerisation of the composition often leads to formation of a material with a volume which is different from and generally less than the volume the composition had prior to the polymerisation. In other words, during polymerisation expansion or more frequently contraction may occur. In cases where the composition is applied in an adhesive material, which is for instance meant to fill a gap between two surfaces, contraction for example leads to shrinkage stresses in this material once polymerisation occurs and the material remains adherent to the two surfaces. Often, also visco-elastic properties or other product properties may change during polymerisation as a result of the newly formed molecular structures. Where the viscous property may have an advantageous, stress relieving effect on the development of polymerisation shrinkage stresses, the elastic properties are mainly causing the stresses due to shrinkage strain. Preferably, the shape of the material is amended during polymerisation to end up with a material in the required shape, with a minimum of stresses.

In the light of the above mentioned problems, there is a need to control the kinetics of the polymerisation and/or the molecular structures formed during the polymerisation. If the polymerisation can temporarily be delayed after a first stage of polymerisation, the shape of the material which still posesses some paste-like, viscous features, can be re-arranged in an attempt to, for instance, minimise shrinkage stresses. In the art, it has been proposed to control the kinetics of the polymerisation by means of controlling the amount of energy applied to the composition. However, the amount of energy applied to the composition is rather difficult to adjust and/or to control and hence, the number of ions or radicals produced during the initiators reaction is difficult to control. Consequently, the kinetics of the polymerisation and/or the molecular structures formed during the polymerisation are difficult to control.

It is an object of the invention to provide a method to initiate the polymerisation in a more controllable way than was hitherto possible with polymerisable compositions known from the prior art.

This object is achieved by providing a polymerisable composition comprising at least two mutually different initiators. This allows for controlling initiating the polymerisation by exploiting the differences between the two mutually different initiators.

Preferably, a first one of the at least two mutually different initiators is capable of initiating polymerisation of the composition upon intake of energy with a first predetermined characteristic and a second one of the at least two mutually different initiators is capable of initiating further polymerisation of the composition upon intake of energy with a second predetermined characteristic, wherein the first characteristic and the second characteristic are mutually different.

Hence, it is possible to control initiating the polymerisation by ensuring in a first period of time the intake of energy with the first predetermined characteristic by the first one of the at least two mutually initiators present in the composition. Upon this first activation, ions and/or radicals are produced, and the polymerisation is triggered off. Those skilled in the art will be able to determine by routine experiments a relationship between the amount of the first one of the at least two mutually different initiators needed in the composition to obtain desired kinetics of the polymerisation, and a desired molecular structure and/or a desired change of visco-elastic properties of the composition as a result of the polymerisation.

After having achieved at least desired kinetics, desired visco-elastic properties, or a desired molecular structure, in a second period of time, the intake of energy with the second predetermined characteristic by the second one of the at least two mutually different initiators present in the composition, may be ensured to further control polymerisation. The kinetics of the further polymerisation and/or the further change in visco-elastic properties and/or the molecular structure as further obtained, will be related to the amount of the second one of the at least two mutually initiators present in the composition. Also these relationships may easily be determined by routine experimenting by a man skilled in the art.

One of the advantages is that the control is simply carried out by switching from use of one energy source to use of another energy source. In other words, a user may determine when the second period of time starts and in that sense the user is in control. The second period of time may partly overlap with the first period of time or may have no overlap at all.

Such control of the polymerisation may for instance be advantageous in a situation where the polymerisable composition has, as a result of the use of an energy source in the first period, polymerised up to a certain degree, and it seems at this moment in time necessary to adapt the shape of the material formed by the polymerisation. The kinetics, the molecular structure as obtained so far and/or the visco-elastic properties as obtained so far may enhance stress relief by viscous flow of the polymerisable composition or may still allow for such a correction of shape before another energy source is used in the second period to ensure further polymerisation. After all, the kinetics of the polymerisation are related to the amount of the first one of the at least two mutually initiators present in the composition. Polymerisation may thus occur up to a degree which also corresponds to the amount of the first one present in the polymerisable composition. The molecular structure formed, so far and consequently the visco-elastic changes of the composition accompanying this polymerisation, may accordingly also correspond to the amount of the first one of the at least two mutually different initiators present in the composition. In the second period enough polymerisation may occur to "pin down" the shape of the material as corrected after ordering the first period and/or to realize the requested optimal degree of conversion of the polymerisable composition.

As stated before, the energy may be applied in the form of heat, ultrasound, microwave radiation or any other suitable form.

Preferably however, the energy comprises light. In that case, both the first predetermined characteristic and the second predetermined characteristic may comprise a wavelength characteristic. This ensures a sharp contrast between activation of the first one of the at least two initiators and activation of the second one of the at least two initiators. Hence, the desired control will be very accurate. A user may only have to switch on a light source which produces light with a different wave length, when switching from the first period to the second period.

It is further preferred that the composition comprises a resin based material and even more preferred that the resin based material comprises a dental restorative resin based material. This allows for use of such compositions as a filling for a dental cavity.

Preferably the resin based material comprises polymers out of the group of biologically compatible resins, e.g. resins on the basis of the acrylate or methacrylate and/or derivates thereof such as BisGMA (Bisphenol A diglycidyl ether dimethacrylate), TEGDMA (tri[ethylene glycol] dimethacrylate) , UDMA (urethane dimethacrylate) Bis-EMA(6)1 (Bisphenol A polyethylene glycol diether dimethacrylate) HEMA (2-hydroxyethylmethacrylate), NIPAAm (N-isopropylacrylamide); HEA (2-hydroxyethylacrylate), AA (acrylamide), GMA (glycerylmethacrylate), GA (glycerylacrylate) WPMAAm (hydroxypropylmethacrylamide), DMAEMA (dimethyl-aminoethylmethacrylate) and DMAEA (dimethylaminoethylacrylate) etc. as used frequently in dentistry for resin based restoratives.

At least one of the at least two mutually initiators may comprise camphorquinone based initiators. These are well known initiators and readily available. Also suitable are initiators of the group consisting of α, α-azo isobutyro nitrile (AIBN), and bis (acyl phosphine oxide) (BAPO).

Further the invention relates to a method for polymerising a polymerisable composition comprising at least two mutually different initiators. According to the invention, this method further comprises:
- in a first period of time providing to the composition energy with a first predetermined characteristic; and
- in a second period of time providing to the composition energy with a second characteristic,
wherein the first period of time is different from the second period of time, the polymerisation of the composition occurs upon intake of energy with the first predetermined characteristic by a first one of the at least two mutually different initiators, and further polymerisation of the composition occurs upon intake of energy with the second predetermined characteristic by a second one of the at least two mutually different initiators.

The invention is also related to a method for filling a dental cavity, wherein the method comprises:
- placing into the cavity a polymerisable composition as described above;
- carrying out a method for starting polymerising a polymerisable composition as described above.
Using a method according to the invention, more control of the kinetics of the polymerisation is possible, leading to inter alia less polymerisation shrinkage stresses. On its turn, this allows for re-arranging the shape of the polymerisable composition during the polymerisation.
Although the invention may easily find its use in the field of dentistry, also other fields may benefit from the methods and compositions according to the invention. Especially in restorative actuities where use is made of resin based materials, the invention may be applied to allow for more control of the polymerisation of the composition.

The invention is further illustrated by means of the following nonlimiting example. Percentages are by weight drawn to the total weight of the final composition, unless otherwise indicated.

### EXAMPLE

### Composition:

### Three resin phases are prepared:

(1) BisGMA (Bisphenol A diglycidyl ether dimethacrylate), (2) UDMA (urethane dimethacrylate) and (3) Bis-EMA(6)1 (Bisphenol A polyethylene glycol diether dimethacrylate) are in an amount of approximately 50% independently added to TEGDMA (tri[ethylene glycol] dimethacrylate) as diluent (approximately 40%).

### Filler phase:

Reinforcing silanated glass (ceramic) fillers with an average size of 0.7 µm and a loading of approximately 80%, being partly radiopaque, are added in an amount of 7.8% to the three different resin phases.

### Light sensitive initiators:

Camphorquinone (sensitive to light with a wavelength in the range of 470 nm) (0.5%); and
BAPO (sensitive to light with a wavelength in the range of 430 nm) (0.2%) are also added to the three compositions.

A so-called soft start is reached by illuminating the three polymerisable compositions with light from a light source of a wavelength of 430 nm. The low concentration BAPO was activated resulting in a slow start of the polymerisable composition. During this reaction a maximum benefit of the viscous stage during curing is obtained resulting in the maximum compensation of polymerisation shrinkage by viscous flow instead of the deformation of stresses. Thereafter a maximum degree of cure (conversion) is reached by illuminating the polymerisable composition with a light source of 470 nm activating the higher concentration of the camphorquinone based initiator.

## Claims

1. Polymerisable composition comprising at least two mutually different initiators.

2. Polymerisable composition according to claim 1, wherein a first one of the at least two mutually initiators is capable of initiating polymerisation of the composition upon intake of energy with a first predetermined characteristic and wherein a second one of the at least two mutually different initiators is capable of further initiating polymerisation of the composition upon intake of energy with a second predetermined characteristic, wherein the first characteristic and the second characteristic are mutually different.

3. Polymerisable composition according to claim 2, wherein energy comprises light.

4. Polymerisable composition according to claim 2 or claim 3, wherein both the first predetermined characteristic and the second characteristic comprise a wavelength characteristic

5. Polymerisable composition according to anyone of the preceding claims, wherein the composition further comprises a resin based material.

6. Polymerisable composition according to claim 5, wherein the resin based material comprises a dental restorative resin based material.

7. Polymerisable composition according to claim 5 or 6, wherein the resin based material comprises polymers out of the group of biologically compatible resins, e.g. polymers based on acrylate or methacrylate monomers or derivatives thereof, such as BisGMA, TEGDMA and HEMA.

8. Polymerisable composition according to any one of the previous claims wherein the at least two mutually initiators comprise camphorquinone based initiators and bis (lacylphosphine oxide) type initiators.

9. Method for polymerising a polymerisable composition comprising at least two mutually different initiators, wherein the method comprises:
(a) in a first period of time providing to the composition energy with a first predetermined characteristic; and
(b) in a second period of time providing to the composition energy with a second characteristic,
wherein the first period of time is different from the second period of time, wherein polymerisation of the composition occurs upon intake of energy with the first predetermined characteristic by a first one of the at least two mutually different initiators and wherein further polymerisation of the composition occurs upon intake of energy with the second predetermined characteristic by a second one of the at least two mutually different initiators.

10. Method for filling a dental cavity wherein the method comprises:
(a) placing into the cavity a polymerisable composition according to any one of the claims 1-9, and
(b) carrying out a method for polymerising a polymerisable composition as described in claim 9.
